(19) **Europäisches Patentamt / European Patent Office / Office européen des brevets**

(11) **EP 3 071 301 B1**

(12) **FASCICULE DE BREVET EUROPEEN**

(45) Date de publication et mention de la délivrance du brevet:
**10.01.2018 Bulletin 2018/02**

(51) Int Cl.:
**A61Q 19/08** *(2006.01)*   **A61K 8/97** *(2017.01)*
**A61K 9/00** *(2006.01)*   **A61K 36/49** *(2006.01)*

(21) Numéro de dépôt: **14809498.0**

(22) Date de dépôt: **19.11.2014**

(86) Numéro de dépôt international:
**PCT/FR2014/052962**

(87) Numéro de publication internationale:
**WO 2015/075377 (28.05.2015 Gazette 2015/21)**

(54) **PRINCIPE ACTIF À BASE D'UN EXTRAIT VÉGÉTAL DE CHÊNE À EFFET ANTI-VIEILLISSEMENT CUTANÉ ET COMPOSITION COSMÉTIQUE LE COMPRENANT**

EICHENEXTRAKT ALS ALTERUNGSSCHUTZMITTEL IN KOSMETISCHEN ZUBEREITUNGEN

QUERCUS PLANT EXTRACT AS ACTIVE AGENT WITH SKIN ANTI-AGEING PROPERTIES AND COSMETIC COMPOSITIONS CONTAINING IT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.11.2013 FR 1361417**

(43) Date de publication de la demande:
**28.09.2016 Bulletin 2016/39**

(73) Titulaire: **Charlois Cosmetiques
58700 Murlin (FR)**

(72) Inventeurs:
- **CHARLOIS, Sylvain
  F-92200 Neuilly-sur-Seine (FR)**
- **RIBOULET, Jean-Michel
  F-31170 Tournefeuille (FR)**
- **BREGOU, Muriel
  F-75007 Paris (FR)**

(74) Mandataire: **Ipside
6, Impasse Michel Labrousse
31100 Toulouse (FR)**

(56) Documents cités:
**FR-A1- 2 841 133      FR-A1- 2 921 834
JP-A- 2006 249 018    JP-A- 2011 121 920
KR-A- 20080 097 314**

- **ALMEIDA ISABEL F ET AL: "Oak leaf extract as topical antioxidant: free radical scavenging and iron chelating activities and in vivo skin irritation potential.", BIOFACTORS (OXFORD, ENGLAND) 2008, vol. 33, no. 4, 2008, pages 267-279, XP002729745, ISSN: 0951-6433**
- **A BERGHI ET AL: "253 Quercus suber cork extract displays tensor and smoothing effect on human skin: in vivo study A novel therapy for tumors of epithelial origin", THE JOURNAL OF INVESTIGATIVE DERMATOLOGY, vol. 122, no. 3, 1 mars 2004 (2004-03-01), - 1 mai 2004 (2004-05-01), page A43, XP055140048, GB ISSN: 0022-202X, DOI: 10.1111/j.1523-1747.2004..x**

**Description**

La présente invention s'inscrit dans le domaine de la lutte contre le vieillissement cutané. Plus particulièrement, elle concerne un principe actif cosmétique ou pharmaceutique à base d'un extrait végétal à propriétés antioxydantes, une composition cosmétique ou dermatologique ou pharmaceutique contenant un tel principe actif, et un procédé de traitement cosmétique de la peau mettant en oeuvre une telle composition.

Le vieillissement, correspondant à un déclin progressif des différentes fonctions physiologiques de l'organisme, est un processus complexe se traduisant en particulier, au niveau de la peau, par l'apparition de rides, le relâchement des tissus tant cutanés que sous-cutanés, une perte de l'élasticité la peau, etc. La prévention et/ou le ralentissement du vieillissement cutané sont une préoccupation pour de nombreux individus, et par voie de conséquence un objectif majeur dans le domaine cosmétique.

Une des causes bien connues du vieillissement cutané est la diminution progressive de la capacité des cellules de la peau à lutter contre les phénomènes d'oxydation, et plus particulièrement contre les radicaux libres générés à la suite de certaines agressions extérieures, par exemple un rayonnement ultraviolet auquel la peau est exposée. Il a ainsi été proposé par l'art antérieur, pour améliorer l'aspect de la peau, des principes actifs cosmétiques à effet antioxydant, notamment d'origine végétale, aptes à piéger les radicaux libres et à prévenir de ce fait le stress oxydatif de la peau.

Le document JP 2006 249018 divulgue un agent actif cosmétique obtenu par extraction de bois de chêne. Le document KR 2008 0097314 et la publication d'Almeida et al., dans Biofactors, 2008, vol. 33, pp 267-279, décrivent des compositions cosmétiques contenant en tant qu'agent actif un extrait obtenu à partir de feuilles de chêne.

Il a maintenant été découvert par les présents inventeurs, de manière tout à fait inattendue, qu'un extrait végétal particulier, obtenu par extraction de matières premières végétales issues du chêne, présente un pouvoir antioxydant particulièrement important, qui fait de lui un principe actif cosmétique particulièrement efficace pour lutter contre le vieillissement cutané. Ce principe actif présente notamment un effet antirides important, ainsi en particulier qu'une action anti-collagénase, une action anti-tyrosinase et une action anti-hyaluronidase. Il a également été découvert par les présents inventeurs que ce principe actif présentait des propriétés particulièrement avantageuses pour le domaine dermatologique ou pharmaceutique, en particulier un effet prévenant l'agrégation plaquettaire et un effet anti-inflammatoire, permettant notamment d'atténuer les oedèmes.

Un premier objet de la présente invention est ainsi un principe actif cosmétique ou pharmaceutique comprenant un extrait végétal obtenu par extraction d'un mélange de matières premières végétales, ce mélange contenant au moins de l'écorce de chêne, du bois de chêne et des feuilles de chêne.

Ce mélange de matières premières végétales peut en outre comporter des bourgeons de chêne.

On ne préjugera pas ici des mécanismes sous-tendant l'obtention de l'effet antioxydant élevé du principe actif selon l'invention. Cependant, une analyse d'extraits végétaux conformes à l'invention démontre une teneur élevée en polyphénols, qui pourrait expliquer au moins en partie cet effet. Par exemple, la teneur en polyphénols totaux de tels extraits obtenus à partir de bois, feuilles et écorce de chêne, déterminée selon la méthode « 2.8.14. Détermination des tanins dans les drogues végétales » de la Pharmacopée Européenne 7.5, a révélé une teneur en polyphénols totaux pouvant aller jusqu'à 3,1 grammes par kilogramme de matières premières. Une analyse plus fine a révélé que ces extraits contiennent des polyphénols, du kaempférol, de la quercétine, de l'acide gallique et de l'acide ellagique.

En outre, et de manière tout à fait surprenante, il a été découvert par les présents inventeurs que le principe actif selon l'invention présente une activité anti-tyrosinase particulièrement élevée, et par voie de conséquence une forte action éclaircissante sur la peau.

Il présente en outre également une forte activité anti-hyaluronidase.

Préférentiellement, l'extrait végétal est un extrait aqueux, pouvant par exemple, mais non limitativement, être obtenu par infusion des matières premières végétales dans un véhicule aqueux, selon une technique classique en elle-même. Un tel mode d'obtention, ne mettant en oeuvre aucun solvant organique, s'avère tout à fait avantageux pour les applications du principe actif selon l'invention dans les domaines de la cosmétique, de la dermatologie et de la pharmacie.

A titre d'exemple, l'extrait végétal entrant dans la composition du principe actif selon l'invention peut être obtenu par immersion du mélange de matières premières végétales avec de l'eau à ébullition, par exemple dans un rapport massique eau / mélange de matières végétales compris entre 6/1 et 9/1, pendant une durée de quelques heures, par exemple d'une heure. Autrement, la température de l'eau mise en oeuvre pour réaliser l'extraction peut être inférieure, notamment comprise entre 50 et 70 °C environ, par exemple comprise entre 55 et 65 °C.

Selon une caractéristique préférentielle particulièrement avantageuse de l'invention, l'extrait végétal est un extrait susceptible d'être obtenu par extraction du mélange de matières premières végétales par un véhicule d'extraction contenant de l'eau et de la glycérine, la glycérine étant notamment présente dans le véhicule dans une quantité en poids supérieure à celle de l'eau.

La température de ce véhicule d'extraction peut notamment être comprise entre 50 et 70 °C environ, par exemple comprise entre 55 et 65 °C.

La durée d'extraction peut par exemple être comprise entre 1 et 3 jours.

Le rapport massique véhicule d'extraction / mélange de matières végétales peut être sensiblement égal à 10.

Le mélange de matières premières végétales peut contenir des quantités massiques sensiblement égales de chaque constituant issu du chêne. Le mélange de matières premières végétales contient les concentrations en matières premières végétales suivantes, exprimées en masse par rapport à la masse totale du mélange :

- 20 à 80 % de feuilles de chêne,
- 20 à 60 % de bois de chêne,
- et le cas échéant, 20 à 60 % d'écorce de chêne.

Dans des variantes le mélange de matières premières végétales contient les concentrations en matières premières végétales suivantes, exprimées en masse par rapport à la masse totale du mélange :

- 5 à 50 % de feuilles de chêne,
- 5 à 50 % de bois de chêne,
- et 30 à 90 % d'écorce de chêne.

Dans des modes de réalisation particulièrs notamment en terme d'activité antioxydante, le bois, l'écorce et les feuilles de chêne sont présents dans le mélange de matières premières végétales soumis à extraction en quantités massiques sensiblement égales. Le mélange de matières premières végétales contient les concentrations en matières premières végétales suivantes, exprimées en masse par rapport à la masse totale du mélange :

- 5 à 15 %, par exemple environ 10 %, de feuilles de chêne,
- 5 à 15 %, par exemple environ 10 %, de bois de chêne,
- et 70 à 90 %, par exemple environ 80 %, d'écorce de chêne.

De telles proportions de chacun des constituants du mélange initial de matières premières végétales soumis à extraction s'avèrent particulièrement avantageuses en termes de performance antioxydantes du principe actif selon l'invention. De manière tout à fait inattendue, l'effet synergique de ces constituants y est particulièrement important.

Le chêne peut être choisi parmi toute espèce ou mélange d'espèces existantes. Préférentiellement, on utilise, en tant que matières premières végétales soumises à extraction pour obtenir le principe actif selon l'invention, un mélange de matières premières issues de chêne d'une espèce du genre *Quercus,* ou d'un mélange de chênes appartenant à différentes espèces du genre *Quercus.*

Par exemple, le mélange de matières premières végétales est issu de chêne appartenant à une espèce choisie parmi *Quercus robur* (communément appelé chêne pédonculé), *Quercus petraea* (ou *Quercus sessiflora,* communément appelé chêne sessile ou chêne rouvre) et *Quercus x rosacea, Quercus pyrenaica,* ou d'un mélange de chênes de telles espèces. *Quercus x rosacea* est une espèce hybride des espèces *Quercus robur* et *Quercus petraea,* qui coexiste avec ces espèces dans de nombreuses forêts.

Le bois de chêne peut être du bois frais, c'est-à-dire du bois non séché, ou du bois ayant été soumis à une étape de séchage partiel, voire total.

Préférentiellement, le bois de chêne est du bois frais partiellement séché, qui présente moins de risques de contamination, ainsi que l'avantage d'une meilleure reproductibilité de composition du principe actif.

Dans le principe actif selon l'invention, l'extrait végétal peut être associé à tout autre composant, sous réserve qu'un tel composant n'inhibe pas l'action de l'extrait, notamment son effet anti vieillissement et en particulier son effet antioxydant. Par exemple, l'extrait végétal peut être associé à un ou plusieurs agents conservateurs, classiques en eux-mêmes dans le domaine cosmétique et/ou pharmaceutique, par exemple un mélange d'acide citrique, de benzoate de sodium et de sorbate de potassium.

Dans des variantes très différentes de l'invention, l'extrait végétal est obtenu par extraction d'un mélange de matières premières végétales, ce mélange contenant au moins deux constituants choisis parmi le bois de chêne, les feuilles de chêne et les bourgeons de chêne.

Dans des modes de réalisation particuliers de l'invention, le mélange de matières premières végétales contient au moins du bois de chêne et des feuilles de chêne.

Ce mélange de matières premières végétales peut en outre comporter de l'écorce de chêne.

Un deuxième objet de l'invention est l'utilisation d'un principe actif selon l'invention, répondant à l'une ou plusieurs des caractéristiques ci-avant, dans le domaine cosmétique, pour lutter contre le vieillissement cutané.

La présente invention concerne également l'utilisation d'un principe actif selon l'invention, répondant à l'une ou plusieurs des caractéristiques ci-avant, pour la fabrication d'une composition cosmétique ou dermatologique ou pharmaceutique à usage topique.

Un autre aspect de l'invention est une composition cosmétique ou dermatologique ou pharmaceutique à usage topique, qui comporte un principe actif selon l'invention, répondant à l'une ou plusieurs des caractéristiques ci-avant, dans un excipient cosmétiquement ou pharmaceutiquement compatible.

Par l'expression « excipient cosmétiquement compatible », on entend que l'excipient est adapté à une utilisation au contact de cellules humaines et animales, en particulier de cellules de la peau. De préférence, cet excipient présente une odeur, une couleur et un toucher agréables, il ne génère pas d'inconforts inacceptables susceptibles de détourner un utilisateur de la composition selon l'invention.

Outre le principe actif conforme à l'invention, tel qu'il a été décrit ci-avant, la composition peut contenir un ou plusieurs ingrédient(s) actif(s) supplémentaires, notamment à propriétés bénéfiques pour la peau, dont l'effet peut le cas échéant s'exercer en synergie avec celui du principe actif selon l'invention.

La composition peut également comporter un ou plusieurs additif(s) classique(s) dans le domaine cosmétique, dermatologique ou pharmaceutique. Par exemple, pour le domaine cosmétique, elle peut contenir un ou des parfums, agents conservateurs, émulsifiants, etc.

Dans des modes de réalisation particuliers de l'invention, la composition contient le principe actif conforme à l'invention dans une teneur comprise entre 0,1 et 10 %, de préférence entre 0,1 et 7 %, par exemple entre 0,1 et 5 %, et par exemple encore entre 0,1 et 2 %, ou entre 2 et 7 % en poids, par rapport au poids total de la composition.

Cette composition peut se présenter sous toute forme classique en elle-même pour des compositions à usage topique, notamment cutané, dans le domaine cosmétique, dermatologique ou pharmaceutique, notamment sous forme d'une crème, d'un lait, d'une lotion, d'un gel, d'un sérum, d'un onguent, etc.

Une telle composition, appliquée sur la peau par voie topique, présente avantageusement une grande efficacité en termes de prévention et/ou ralentissement du vieillissement cutané, principalement du fait de son action protectrice contre le stress oxydatif des cellules de la peau.

Un autre aspect de l'invention est un procédé de traitement cosmétique de la peau, notamment humaine, qui comprend l'application par voie topique sur la peau d'une composition répondant à l'une ou plusieurs des caractéristiques ci-avant. Cette application peut notamment être réalisée une à deux fois par jour, par exemple sur le visage et sur le cou.

Les caractéristiques et avantages de l'invention apparaîtront plus clairement à la lumière des exemples ci-après, fournis à simple titre illustratif et nullement limitatifs de l'invention.

## EXPERIENCE A

EXEMPLE A.1 - Préparation de principes actifs à base d'extraits végétaux obtenus à partir d'un mélange de bois, d'écorce et de feuilles de chêne

A.1.1/ Principe actif E1 - Principe actif à base d'un extrait conforme à l'invention associé à des agents conservateurs

Cet extrait végétal conforme à l'invention est obtenu à partir d'un mélange de bois frais de chêne, d'écorce de chêne et de feuilles de chêne obtenus à partir d'un mélange des espèces suivantes : *Quercus robur, Quercus petraea* et *Quercus x rosacea.* Le bois, les feuilles et l'écorce sont mélangés en parts en poids égales.

1 kg du mélange de matières premières végétales ainsi formé est mis en contact avec de l'eau à ébullition avec 6 kg d'eau, de telle sorte que l'ensemble des matières premières végétales sont en immersion dans le véhicule aqueux.

La durée de l'infusion ainsi réalisée est de 2 heures.

A l'issue de cette étape, le mélange de matières premières végétales est séparé du solvant par plusieurs filtrations successives, puis de la glycérine d'origine végétale est ajoutée à hauteur de 50 % de la masse de produit fini.

Afin de garantir la bonne conservation de cet extrait végétal, des conservateurs (acide citrique, benzoate de sodium, sorbate de potassium) sont additionnés à la concentration de 0,1 % en poids chacun, pour obtenir le principe actif E1 conforme à l'invention.

A.1.2/ Principe actif E2 - Principe actif à base d'un extrait conforme à l'invention dépourvu d'agents conservateurs

Le procédé décrit ci-avant pour le principe actif E1 est appliqué avec les mêmes conditions opératoires. A l'issue de l'extraction, aucun conservateur n'est ajouté à l'extrait végétal obtenu, qui est conservé au froid, plus précisément à 4 °C.

A.1.3/ Principes actifs comparatifs EC1, EC2, EC3

Des principes actifs comparatifs sont réalisés selon le même protocole que décrit ci-avant en référence au principe actif E1, à partir des matières premières suivantes, issues du même mélange de chênes des espèces *Quercus robur, Quercus petraea* et *Quercus x rosacea* :

EC1 : feuilles

EC2 : bois

EC3 : écorce

EXEMPLE A.2 - Analyse de l'activité antioxydante des principes actifs A.2.1 / Test ORAC

Le pouvoir antioxydant du principe actif E1 conforme à l'invention, et des principes actifs comparatifs EC1, EC2 et EC3, est évalué par le test spectrofluorimétrique ORAC (pour l'anglais « Oxygen radical Absorbance Capacity »). De manière générale, le test ORAC consiste en une mesure de la protection exercée par une molécule ou un mélange de molécules donné contre l'oxydation de la fluorescéine par un radical libre stable, le dihydrochlorure de 2,2'-azobis(2-amidino-propane) (AAPH).

La décroissance de la fluorescence est suivie en fonction de la dégradation de la fluorescéine (FL : 3',6'-dihydroxyspiro[isobenzofuran-1 [3H],9'[9H]-3-one]) induite par la décomposition thermique, à 37°C, de l'AAPH en deux radicaux libres (Blois, 1958, Nature, 181: 1199-1200). La présence d'antioxydants dans l'échantillon testé retarde la dégradation de la fluorescence. La méthode est réalisée en microplaques dans lesquelles sont mesurés en parallèle le déclin de la fluorescéine au cours du temps en présence de concentrations croissantes de Trolox, une molécule de référence, analogue structural hydrosoluble de la vitamine E, connu pour son activité antioxydante. Le but est d'obtenir une réponse comparable à celle de la gamme en suivant la cinétique par mesure des aires sous la courbe. Ceci permet, après traitement des données, de calculer l'équivalent Trolox.

Plus particulièrement, les conditions de test sont les suivantes.

La réaction est réalisée dans un tampon phosphate 75 mM à pH 7,4 dans une plaque à 96 alvéoles.

30 μL de principe actif à tester non dilué et 180 μL de solution de fluorescéine (concentration finale 70 nM) sont mélangés et pré-incubés 5 min à 37 °C. Il est ensuite ajouté 90 μL de solution d'APPH (concentration finale 12 nM) et la fluorescence est enregistrée durant 60 min à 485 nm pour la longueur d'onde d'excitation et à 520 nm pour l'émission. Un blanc et 6 concentrations de Trolox (0,1 à 4 μM de concentration finale) sont testés en même temps que les principes actifs. Les échantillons sont tripliqués.

L'aire sous la courbe est calculée pour chaque échantillon par intégration de la courbe de fluorescence et en soustrayant l'aire sous la courbe du blanc. L'équation de régression entre l'aire sous la courbe des échantillons et la concentration du Trolox est déterminée et les valeurs ORAC sont ainsi exprimées en équivalent concentration Trolox.

Les résultats obtenus sont indiqués dans le tableau 1 ci-dessous.

Tableau 1 - Valeurs ORAC obtenues pour les différents principes actifs

| Principe actif | Valeur ORAC (μmol Te/kg) |
|---|---|
| E1 | 15870 ± 3174 |
| EC1 | 11380 ± 2276 |
| EC2 | 10642 ± 2128 |
| EC3 | 5002 ± 1000 |

On y observe que la valeur ORAC obtenue pour le principe actif E1 selon l'invention est élevée. Cette valeur est en outre nettement plus élevée que celles obtenues pour les principes actifs comparatifs EC1, EC2, EC3, à quantités égales de matières premières végétales soumises à extraction. Ceci témoigne, d'une part, d'une activité antioxydante importante du principe actif conforme à l'invention, et, d'autre part, d'une synergie entre les composés extraits des feuilles, du bois et de l'écorce de chêne, pour cet effet antioxydant.

A.2.2/ Test DPPH

Par ailleurs, le pouvoir antioxydant des principes actifs E1 et E2 conformes à l'invention est évalué par le test spectrophotométrique DPPH.

Le test est basé sur la dégradation du radical 1,1-diphényl-2-pycrilhydrazil (DPPH) qui possède un électron non apparié sur un atome du pont d'azote. Du fait de cette délocalisation, les molécules du radical ne forment pas des dimères, c'est-à-dire que le DPPH reste dans sa forme monomère relativement stable à température ordinaire. Un composé antioxydant présent dans l'échantillon testé aura la capacité de donner un électron singulet au radical synthétique DPPH de coloration violette, pour le stabiliser en DPPH de coloration jaune-verte mesurable par spectrophotométrie à 515-518 nm. La présence d'antioxydants dans l'échantillon testé provoque ainsi la décroissance de la coloration violette (Dudonné et al, 2011, J. Agric. Food Chem., 59: 4527-4536) : la mesure de l'efficacité d'un antioxydant s'effectue en suivant la diminution de la coloration violette qui est mesurée au cours du temps par le pourcentage de coloration perdue. La méthode est standardisée par rapport au TROLOX.

Plus précisément, les conditions opératoires sont les suivantes.

La méthode utilise une réaction sur un mélange de 1 mL de solution de DPPH avec 4 mL de principe actif à tester dilué au 1/50ème. L'acide ascorbique est employé comme standard. Les échantillons sont tripliqués.

L'absorbance est mesurée à 515 nm après 10 min de réaction et une courbe étalon est construite.

Les résultats obtenus, exprimés en % d'inhibition de l'activité du DPPH, sont indiqués dans le tableau 2 ci-dessous.

Tableau 2 - résultats du test de détermination de l'activité antioxydante DPPH des principes actifs E1 et E2 conformes à l'invention

|  | Test DPPH (% d'inhibition) |
|---|---|
| Principe actif E1 | 62,7 $\pm$ 1,5 |
| Principe actif E2 | 76 $\pm$ 1 |

Là encore, on observe un effet antioxydant important des principes actifs conformes à l'invention.

EXEMPLE A.3 - Test de cytotoxicité

Un test de cytotoxicité est réalisé sur des kératinocytes épidermiques (NHEK) pour le principe actif E2 conforme à l'invention.

Les concentrations suivantes sont testées : 0,1 ; 0,3 ; 0,5 ; 1 ; 3 % (p/p) (% en poids de principe actif à tester, par rapport au poids final de milieu de culture contenant le principe actif).

A cet effet, les solutions de principe actif aux concentrations ci-dessus sont diluées, à différents facteurs de dilution, dans une solution stock constituée de 50 % d'eau ultra pure et de 50 % de glycérine. Chacune des solutions ainsi obtenues est ajoutée à du milieu de culture Keratinocyte-SFM, et des cellules sont cultivées dans le milieu ainsi obtenu pendant 72 heures, à 37 °C et sous 5 % de $CO_2$.

A la fin du traitement, les cellules sont incubées en présence de sel de tétrazolium MTT (bromure de 3-(4,5-diméthylthiazol-2-yl)-2,5-diphényl tétrazolium), dont la transformation en cristaux bleus de formazan est proportionnelle à l'activité de la succinate déshydrogénase, une enzyme mitochondriale. Après dissociation des cellules et solubilisation du formazan par ajout de diméthylsulfoxyde (DMSO), la densité optique, représentative du nombre de cellules vivantes et de leur activité métabolique, est mesurée avec un lecteur de microplaques à 540 nm (VersaMax®, Molecular Devices).

Des témoins non traités (NT) et traités avec la solution stock seule, à différentes dilutions dans le milieu de culture, sont également réalisés.

Chaque expérience est tripliquée.

Les résultats obtenus, pour le principe actif conforme à l'invention à une concentration de 0,5 %, sont indiqués dans le tableau 3 ci-après.

Tableau 3 - Test de cytotoxicité sur kératinocytes épidermiques pour le principe actif E2 conforme à l'invention à une concentration de 0,5 %

|  | NT | Solution stock | | | | | E2 à 0,5 % | | |
|---|---|---|---|---|---|---|---|---|---|
| Concentration dans le milieu de culture | - | 0,0048 | 0,014 | 0,041 | 0,123 | 0,37 | 1,11 | 3,33 | 10 |
| Viabilité (%) | 92 | 91 | 107 | 99 | 89 | 89 | 68 | 43 | 8 |
|  | 103 | 110 | 106 | 100 | 95 | 83 | 71 | 52 | 9 |
|  | 104 | 106 | 104 | 104 | 94 | 85 | 83 | 53 | 9 |
| Moyenne | 100 | 102 | 106 | 101 | 93 | 86 | 74 | 49 | 9 |
| esm | 3 | 6 | 1 | 2 | 2 | 2 | 5 | 3 | 0 |

(suite)

| | NT | Solution stock | | | | | E2 à 0,5 % | | |
|---|---|---|---|---|---|---|---|---|---|
| Observations morphologiques | + | + | + | + | + | + | ± | -, * | -,* |

esm = erreur standard de la moyenne
NT = témoin non traité
+ = population normale, ± = réduction de croissance ; - = toxicité, * = altérations morphologiques

Ces résultats démontrent une très faible cytotoxicité du principe actif E2 selon l'invention sur les kératinocytes. La concentration induisant 50% de mort cellulaire (CT50) est évaluée à 2,16 %.

EXEMPLE A.4 - Activité sur le peroxyde d'hydrogène

Le principe actif E2 conforme à l'invention est soumis au test suivant, à des concentrations de 0,1 ; 0,3 ; 0,5 ; 1 ; 3 % p/p (% en poids de principe actif à tester, par rapport au poids final de milieu contenant le principe actif).
L'essai est réalisé au moyen du kit de dosage Amplex® Red Hydrogen Peroxyde/Peroxydase Assay Kit (Molecular Probes®), dont le réactif 10-acétyl-3,7-dihydroxyphénoxazine est utilisé pour déterminer la quantité de peroxyde d'hydrogène présent. Le substrat non coloré réagit avec le peroxyde d'hydrogène ($H_2O_2$) dans un rapport stoechiométrique 1:1 pour produire de la résofurine hautement fluorescente. (Anal Biochem 253, 162 (1997) ; J Immunol Methods 202, 133 (1997) ; J Neurochem 79, 266 (2001) ; Am. J Physiol Lung Cell Mol Physiol 281,L993 (2001); Mol Hum Reprod 7, 237 (2001); Anal Biochem 287, 196 (2000) ; J Invest Dermatol 112, 751 (1999)).
Le principe actif selon l'invention et le BHA (3-tert-butyl-4-hydroxyanisole), en tant que témoin, à une concentration de 50 $\mu$M, sont incubés en présence d'$H_2O_2$, de réactif Amplex® Red et d'enzyme Horse Radish Peroxydase (HRP) pendant 30 min à température ambiante. Puis la fluorescence est mesurée, pour une longueur d'onde d'excitation de 540 nm et une longueur d'onde d'émission de 590 nm.
Des contrôles d'auto-fluorescence et d'absorption de fluorescence (quenching) sont aussi réalisés pour chaque concentration de principe actif selon l'invention et du témoin. Les contrôles d'absorption de fluorescence sont réalisés en incubant le peroxyde d'hydrogène ($H_2O_2$), le réactif Amplex® Redet l'enzyme HRP pendant 30 min. Le principe actif selon l'invention et le témoin sont ensuite ajoutés à la fin de la réaction, avant mesure de la fluorescence. Une diminution de la fluorescence émise dans ce cas correspond à une absorption de photons et non à une inhibition spécifique.
Le bruit de fond des produits seuls (contrôle d'interférence) est retranché aux valeurs brutes obtenues. Chaque expérience est tripliquée.
Les résultats obtenus, en termes de pourcentage d'inhibition du peroxyde d'hydrogène, sont montrés dans le tableau 4 ci-après.

Tableau 4 - Dosage du peroxyde d'hydrogène pour le principe actif E2 conforme à l'invention

| Composé | Concentration | % inhibition | esm | p[(1)] | Intensité de fluorescence (UA) | % quenching |
|---|---|---|---|---|---|---|
| Témoin non traité | | 0 | 1 | - | 3046 | 0 |
| BHA | 50 $\mu$M | 60 | 1 | *** | 3007 | 1 |
| E2 | 0,1% | 58 | 1 | *** | 3083 | 0 |
| | 0,3% | 72 | 3 | *** | 2929 | 4 |
| | 0,5% | 74 | 1 | *** | 2736 | 10 |
| | 1% | 81 | 1 | *** | 2395 | 21 |
| | 3% | 79 | 1 | *** | 1375 | 55 |

esm = erreur standard de la moyenne
(1) seuil de significativité statistique; *** = <0,001, extrêmement significatif

Ces résultats démontrent que le principe actif E2 conforme à l'invention diminue fortement et significativement la quantité de peroxyde d'hydrogène libre dans le milieu testé (contenant le tampon, le réactif et le principe actif) révélé par la réaction Amplex® Red, pour toutes les concentrations testées. L'effet maximal est observé dès la concentration de 0,3 %.
L'action constatée du BHA, qui était attendue, permet de valider cet essai.

EXEMPLE A.5 - Activité de la hyaluronidase

Le principe actif E2 conforme à l'invention est soumis au test suivant, à des concentrations de 0,1 ; 0,3 ; 0,5 ; 1 ; 3 % p/p (% en poids de principe actif à tester, par rapport au poids final de milieu contenant le principe actif).

Le substrat est l'acide hyaluronique, testé à 0,04 % en tampon phosphate de sodium.

L'enzyme est la hyaluronidase (EC 3.2.1.35) testée à 10 U/ml en tampon phosphate de sodium / chlorure de sodium / BSA.

Le principe actif selon l'invention et les témoins (vitamine C à 10 mg/l et palmitate d'ascorbyle à 200 µg/ml) sont mis en présence de la hyaluronidase et incubés pendant 10 min à 37 °C sous agitation. L'acide hyaluronique est ensuite ajouté et les mélanges réactionnels sont incubés à 37 °C pendant 2 h. Des conditions contrôles additionnelles sont réalisées en incubant le produit et le substrat en l'absence de l'enzyme, ou en incubant le produit seul correspondant à un contrôle interférence. La réaction enzymatique est ensuite stoppée par précipitation de l'acide hyaluronique avec une solution d'albumine de sérum bovin (BSA). L'absorption (DO) à 540 nm est mesurée à l'aide d'un lecteur de microplaques (ThermoMax, Molecular Devices).

Chaque expérience est tripliquée.

Les résultats sont exprimés en pourcentage d'activité de la hyaluronidase et en pourcentage d'inhibition de l'activité de la hyaluronidase.

L'IC$_{50}$, ou concentration inhibitrice médiane, est calculée pour chaque échantillon testé.

Les résultats obtenus sont montrés dans le tableau 5 ci-après.

Tableau 5 - Inhibition de l'activité de la hyaluronidase par le principe actif E2 conforme à l'invention

| Composé | Concentration | Inhibition hyaluronidase (%) | esm (%) | p[1] | IC$_{50}$ estimée (%) |
|---|---|---|---|---|---|
| Témoin non traité | - | 0 | 0 | - | - |
| Vitamine C | 10 mg/ml | 71 | 3 | *** | - |
| Palmitate d'ascorbyle | 200 µg/ml | 80 | 1 | *** | - |
| E2 | 0,1% | 6 | 0 | *** | 0,77 |
| | 0,3% | 16 | 1 | *** | |
| | 0,5% | 28 | 5 | ** | |
| | 1% | 64 | 1 | *** | |
| | 3% | 91 | 1 | *** | |

esm = erreur standard de la moyenne
(1) seuil de significativité statistique; *** = <0,001, extrêmement significatif; ** = 0,001 à 0,01, très significatif

Ces résultats démontrent que le principe actif E2 conforme à l'invention inhibe fortement, et de manière dépendante de la concentration, l'activité de la hyaluronidase.

L'effet constaté des témoins vitamine C et palmitate d'ascorbyle, qui était attendu, permet de valider l'essai.

EXEMPLE A.6 - Activité des métallo protéases matricielles (MMP) totales

Les enzymes appelés métallo protéases matricielles sont naturellement présentes dans la peau et sont à l'origine de la dégradation des fibres de collagène.

Le principe actif E2 conforme à l'invention est soumis au test suivant, à des concentrations de 1 et 3 % p/p, selon le protocole décrit dans la publication de Sim et al., 2007, Arch Pharm Res, 30(3): 290-298.

Les MMP totales (obtenues selon un protocole standard à partir de surnageants de fibroblastes dermiques humains normaux, irradiés avec des UVB (20 mJ/cm$^2$)) sont traitées par le mélange inflammatoire acétate de phorbol myristate (PMA) et facteur de nécrose tumorale (TNFα).

Le substrat fluorescent est le peptide fluorogénique décrit dans la publication de Lee et al., 1999, Annals of the New York Academy of Sciences, 878: 635-637, et commercialisé sous la référence M6412 par la société Sigma-Aldrich.

Le principe actif E2 conforme à l'invention ou le témoin (O-phénantroline testée à 0,03; 0,1 ; 0,3 et 1 mM) sont préincubés pendant 10 min sur glace en présence des MMP totales activées, puis le substrat fluorescent est ajouté. Les plaques ont été incubées pendant 3h à 37°C. Un contrôle d'interférence du composé (composé en présence du mélange réactionnel sans enzyme) et un contrôle « quenching » (mélange réactionnel contenant le substrat et l'enzyme

EP 3 071 301 B1

dans lequel le composé n'est ajouté qu'en fin d'incubation) ont été réalisés en parallèle. Le contrôle interférence permet de vérifier l'absorption non spécifique de la fluorescence par le composé et donc la spécificité de l'inhibition observée dans la condition test correspondante.

Chaque expérience est tripliquée.

A la fin de l'incubation, la fluorescence produite suite à la dégradation du substrat est mesurée par fluorimétrie pour une longueur d'onde d'excitation de 328 nm et une longueur d'onde d'émission de 393 nm, au moyen du dispositif SpectroMax Gemini, Molecular Devices.

Le % d'inhibition des MMPs totales est calculé pour chaque échantillon testé. Les résultats obtenus sont montrés dans le tableau 6 ci-après.

Tableau 6 - Inhibition de l'activité des MMP totales par le principe actif E2 conforme à l'invention

| Composé | Concentration | % inhibition | esm | p[(1)] | Quenching (%) |
|---|---|---|---|---|---|
| Témoin non traité | - | 0 | 1 | - | 0 |
| O-Phénanthroline | 0,03 mM | 1 | 0 | ns | 3 |
| | 0,1 mM | 15 | 1 | *** | 7 |
| | 0,3 mM | 73 | 0 | *** | 11 |
| | 1 mM | 97 | 0 | *** | 27 |
| E2 | 1 % | 15 | 0 | *** | 5 |
| | 3 % | 35 | 0 | *** | 9 |
| esm = erreur standard de la moyenne (1) seuil de significativité statistique; *** = <0,001, extrêmement significatif; ns > 0,05, non significatif | | | | | |

Ces résultats démontrent que le principe actif E2 conforme à l'invention inhibe significativement, et de manière dépendante de la concentration, l'activité des MMP totales.

L'effet constaté des témoins O-phénanthroline, qui était attendu, permet de valider l'essai.

Les résultats des tests ci-avant démontrent clairement que le principe actif E2 conforme à l'invention présente, aux plus fortes concentrations (3 % et 1 %), une activité anti-radicalaire importante, une inhibition forte de l'activité de la hyaluronidase et une inhibition significative de l'activité des métallo protéases matricielles. Aux plus faibles concentrations (0,5 %, 0,3 % et 0,1 %), il présente également une activité anti-radicalaire importante et une inhibition significative de l'activité de la hyaluronidase.

Le principe actif E1, qui ne diffère du principe actif E2 que par la présence d'agents conservateurs, présente les mêmes propriétés avantageuses, dans la mesure où cette présence d'agents conservateurs n'influe nullement sur ces propriétés.

EXEMPLE A.7 - Composition cosmétique

Une composition cosmétique conforme à l'invention, sous forme d'une crème de jour, comprend les ingrédients suivants, dans les quantités en poids suivantes :

| | |
|---|---|
| Eau purifiée | QSP 100 |
| Principe actif E1 | 0,5 à 3 % |
| Acétate de Vitamine E | 0,2 à 1 % |
| Phytosqualane | 1 à 5 % |
| Beurre de karité | 1 à 3 % |
| Glycérine | 5 à 10 % |
| Huile de macadamia | 1 à 2 % |
| Triglycérides capriques/capryliques | 0,5 à 2 % |
| Parfum | 0,1 % |

Cette composition est destinée à être appliquée par voie topique sur la peau du visage et du cou, à raison d'une fois par jour, par exemple le matin.

Elle présente un effet antivieillissement cutané important.

**EXPERIENCE B**

EXEMPLE B.1 - Préparation de principes actifs à base d'extraits végétaux obtenus à partir d'un mélange de bois, d'écorce et de feuilles de chêne

B.1.1/ Principe actif E4 - Principe actif à base d'un extrait associé à des agents conservateurs

Cet extrait végétal est obtenu à partir d'un mélange de bois de chêne, d'écorce de chêne et de feuilles de chêne obtenus à partir d'un mélange des espèces suivantes : *Quercus robur, Quercus petraea.* Le bois, les feuilles et l'écorce sont mélangés en parts en poids égales.

10 kg du mélange de matières premières végétales ainsi formé sont utilisés.

Les agents conservateurs (acide citrique, benzoate de sodium, sorbate de potassium) sont dilués dans 50 kg d'eau à une température comprise entre 55 et 65 °C, à la concentration de 0,1 % en poids chacun. La solution obtenue est versée sur le mélange de matières premières végétales.

Le mélange est infusé pendant 48 h en faisant circuler le solvant d'extraction continuellement, puis filtré à l'aide d'un tamis pendant 10 h.

Le filtrat obtenu est filtré stérilement et mélangé à de la glycérine en proportion en poids de 1:1.

B.1.2/ Principes actifs E5, E6 et E7 - Principes actifs à base d'extraits dépourvus d'agents conservateurs

Ces extraits végétaux sont obtenus à partir de mélanges de bois de chêne, d'écorce de chêne et de feuilles de chêne obtenus à partir d'un mélange des espèces suivantes : *Quercus robur, Quercus petraea.*

Le bois, les feuilles et l'écorce sont mélangés dans les proportions en poids suivantes :

Extrait E5 : 80 % écorce, 10 % bois, 10 % feuilles
Extrait E6 : 60 % écorce, 20 % bois, 20 % feuilles
Extrait E7 : 33,33 % écorce, 33,33 % bois, 33,33 % feuilles

Pour chacun de ces extraits, 10 kg du mélange de matières premières végétales sont utilisés.

Le protocole d'obtention des extraits est le suivant.

6 kg d'eau à une température comprise entre 55 et 65 °C, puis 94 kg de glycérine, sont successivement versés sur le mélange de matières premières végétales.

Le mélange est infusé pendant 48 h en faisant circuler le solvant continuellement, puis filtré à l'aide d'un tamis pendant 10 h. Le filtrat obtenu est ensuite filtré stérilement.

B.1.3/ Principes actifs comparatifs EC4, EC5, EC6

Des principes actifs comparatifs sont réalisés selon le même protocole que décrit ci-avant en référence aux principes actifs E5 à E7, à partir pour chacun de 10 kg des matières premières suivantes, issues du même mélange de chênes des espèces *Quercus robur* et *Quercus petraea* :

EC4 : écorce
EC5 : feuilles
EC6 : bois

EXEMPLE B.2 - Analyse de l'activité antioxydante des principes actifs

L'activité antioxydante des principes actifs E4, E5, E6 et E7, et des principes actifs EC4, EC5 et EC6, est mesurée de deux manières différentes :

- par le test ORAC, comme décrit dans l'Expérience A ci-avant,
- par calcul de la quantité de polyphénols totaux (AOP) selon le protocole décrit dans la pharmacopée européenne Ph. Eur. 2.8.14.

Les résultats obtenus sont indiqués dans le tableau 7 ci-après.

Tableau 7 - Résultats de tests d'évaluation de l'activité antioxydante pour des principes actifs E4 à E7 et des principes actifs EC4 à EC6

| Principe actif | Résultat ORAC (μmol Te/kg) | Résultat AOP (g/kg) |
|---|---|---|
| E4 | 25590 | 2,118 |
| E5 | 40410 | 3,1 |
| E6 | 30470 | 2,4 |
| E7 | 32300 | 2,9 |
| EC4 | 38770 | 3,021 |
| EC5 | 22220 | 2,326 |
| EC6 | 22080 | 2,7 |

Les principes actifs E5, E6 et E7, obtenus par extraction au moyen d'un véhicule aqueux contenant un mélange d'eau et de glycérine, s'avèrent plus performants que le principe actif E4.

En outre, l'efficacité antioxydante du principe actif E5 est particulièrement importante, et même supérieure à celle des principes actifs comparatifs EC4, EC5 et EC6, pour des quantités totales de matières premières initiales identiques, et des protocoles d'extraction également identiques. Cette supériorité est avérée quel que soit le test effectué. Elle démontre clairement un effet synergique des différents constituants du mélange de matières premières permettant d'obtenir le principe actif E5 selon l'invention.

EXEMPLE B.3 - Effet sur l'activité enzymatique de la hyaluronidase

Au cours du vieillissement cutané, le stock d'acide hyaluronique, l'un des principaux composants de la matrice extracellulaire, diminue progressivement. Des inhibiteurs de la hyaluronidase, enzyme responsable de la dégradation de l'acide hyaluronique, favorisent le maintien du stock d'acide hyaluronique, et participent de ce fait à une action antivieillissement cutané.

Les principes actifs E5 et E7 sont soumis à une analyse de leur capacité d'inhibition de la hyaluronidase, aux différentes concentrations de 0,5 %, 1 % et 3 % (p/p), selon le protocole suivant.

L'enzyme est la hyaluronidase bovine (EC 3.2.1.35), préparée tampon phosphate de sodium / chlorure de sodium / BSA, pH 7, et utilisée à 10 U/ml.

Le substrat est l'acide hyaluronique, préparé en tampon phosphate de sodium à pH 5,35 et testé à 0, mg/ml.

Les principes actifs selon l'invention et la référence (acide 6-O-palmitoylascorbique, noté APA, testé à 300 μg/ml) sont mis en présence de la hyaluronidase et pré-incubés pendant 10 min à 37 °C sous agitation. L'acide hyaluronique est ensuite ajouté et les mélanges réactionnels sont incubés à 37 °C pendant 2 h. Des conditions contrôles sont réalisées en parallèle en incubant les principes actifs ou la référence en présence du substrat mais en absence de l'enzyme (Contrôle sans enzyme) ou en incubant les principes actifs ou la référence seuls (Contrôle interférence). Il est également réalisé un témoin en incubant l'enzyme seule (Témoin).

La réaction enzymatique est ensuite stoppée par précipitation de l'acide hyaluronique avec une solution tampon contenant de l'albumine de sérum bovin (BSA) à pH 3,75. L'absorption (DO) à 540 nm est mesurée à l'aide d'un lecteur de microplaques (VersaMax®, Molecular Devices). L'activité est inversement proportionnelle à la valeur de DO mesurée.

Chaque expérience est tripliquée.

Les résultats sont exprimés en pourcentage d'activité de la hyaluronidase et en pourcentage d'inhibition de l'activité de la hyaluronidase.

L'activité enzymatique de la hyaluronidase est déterminée par l'équation suivante :

$$\text{Activité enzymatique} = \text{Contrôle sans enzyme} - \text{Activité enzymatique brute}$$

Le pourcentage d'activité de la hyaluronidase est obtenu par l'équation :

$$\text{Activité hyaluronidase (\%)} = (\text{Activité enzymatique}/\text{Moyenne}_{\text{Activité enzymatique Témoin}}) \times 100$$

Le pourcentage d'inhibition de la hyaluronidase est enfin obtenu selon l'équation :

$$\text{Inhibition (\%)} = 100 - \text{Activité hyaluronidase (\%)}$$

Les résultats obtenus, exprimés en pourcentage d'activité de la hyaluronidase et en pourcentage d'inhibition de l'activité de la hyaluronidase, sont montrés dans le tableau 8 ci-après.

Tableau 8 - Inhibition de l'activité de la hyaluronidase par les principes actifs E5 et E7

| Principe actif | Concentration | Activité hyaluronidase (%) | Inhibition hyaluronidase (%) | esm (%) | p[(1)] |
|---|---|---|---|---|---|
| Témoin | - | 100 | 0 | 5 | - |
| APA | 300 $\mu$g/ml | 21 | 79 | 2 | *** |
| E5 | 0,5% | 23 | 77 | 3 | *** |
|  | 1 % | 4 | 96 | 2 | *** |
|  | 3 % | 0 | 100 | 0 | *** |
| E7 | 0,5 % | 36 | 64 | 5 | *** |
|  | 1 % | 15 | 85 | 4 | *** |
|  | 3 % | 3 | 97 | 5 | *** |
| esm = erreur standard de la moyenne (1) seuil de significativité statistique ; *** = <0,001, extrêmement significatif | | | | | |

On observe que la référence acide 6-O-palmitoylascorbique (APA), testée à 300 $\mu$g/ml, inhibe fortement l'activité enzymatique de la hyaluronidase (79 % d'inhibition). Cet effet était attendu et permet de valider l'essai.

Dans les conditions expérimentales de cette étude, les principes actifs E5 et E7, testés à 0,5 %, 1 % et 3 %, inhibent tous deux fortement, et de façon dépendante de la concentration, l'activité enzymatique de la hyaluronidase (inhibitions respectives de 100 % et 97 % à la plus forte concentration testée). Le principe actif E5 semble être légèrement plus efficace que le principe actif E7 aux trois concentrations testées.

Ceci démontre les propriétés anti-âges des principes actifs selon l'invention.

EXEMPLE B.4 - Effet sur l'activité enzymatique de la tyrosinase

La tyrosinase est une enzyme clé dans la synthèse de mélanine. Des inhibiteurs spécifiques de cette enzyme peuvent conduire à une inhibition de la synthèse de mélanine et ainsi diminuer l'apparition de taches de vieillesse, dues à une augmentation de la synthèse de mélanine.

Les principes actifs E5 et E7 sont soumis à une analyse de leur capacité d'inhibition de la tyrosinase, à la concentration de 3 % (p/p), selon le protocole ci-après.

L'enzyme est la tyrosinase extraite de mélanocytes épidermiques humains en tampon d'essai (PBS / Triton® X-100).

Le substrat est la dihydroxy-phényl-alanine L3,4 (L-DOPA), en solution mère à 10 mM en tampon d'essai.

Les principes actifs et la référence (acide kojique testé à quatre concentrations entre 0,0625 et 0,5 mM sont mis en présence de l'extrait enzymatique et incubés pendant 10 min sur glace.

A la fin de l'incubation, le substrat est ajouté (2 mM final) et les mélanges sont incubés pendant 1 h à 37 °C.

L'activité enzymatique est évaluée en mesurant la densité optique (DO) à 540 nm à l'aide d'un lecteur de microplaques (VersaMax®, Molecular Devices). En parallèle, la densité optique est également mesurée sans ajout du substrat afin de détecter de potentielles interférences de la référence ou des composés à chaque concentration testée. Toutes les conditions expérimentales ont été réalisées en triplicat, sauf le contrôle interférence, qui a été réalisé une seule fois.

Le pourcentage d'inhibition de la tyrosinase est déterminé par l'équation suivante :

$$\text{Inhibition (\%)} = 100 - [(\text{Valeur / moyenne du témoin}) \times 100]$$

EP 3 071 301 B1

Les résultats obtenus, exprimés en pourcentage d'inhibition de l'activité de la tyrosinase, sont montrés dans le tableau 9 ci-après.

Tableau 9 - Inhibition de l'activité de la tyrosinase par les principes actifs E5 et E7

| Principe actif | Concentration | Inhibition tyrosinase (%) | esm (%) | p[(1)] |
|---|---|---|---|---|
| Témoin | - | 0 | 0 | - |
| Acide kojique | 0,0625 mM | 3 | 1 | * |
| | 0,125 mM | 13 | 2 | ** |
| | 0,25 mM | 19 | 0 | *** |
| | 0,5 mM | 48 | 1 | *** |
| E5 | 3 % | 17 | 1 | *** |
| E7 | 3 % | 24 | 1 | *** |

esm = erreur standard de la moyenne
(1) seuil de significativité statistique ; *** = <0,001, extrêmement significatif ; ** = 0,001 à 0,01, très significatif ; * = 0,01 à 0,05, significatif

On observe que la référence acide kojique, testée de 0,125 à 0,5 mM, inhibe fortement, et de façon dépendante de la concentration, l'activité enzymatique de la tyrosinase extraite de mélanocytes épidermiques humains normaux, avec une IC$_{50}$ aux environs de 0,5 mM. Cet effet était attendu et permet de valider l'essai.

Dans les conditions expérimentales de ce test, les principes actifs E5 et E7, testés à 3 %, inhibent l'activité enzymatique de la tyrosinase de manière significative.

Ceci reflète un effet de diminution de l'apparition des taches de vieillesse (dues à une augmentation de la synthèse de mélanine) des principes actifs selon l'invention.

EXEMPLE B.5 - Composition cosmétique

Une composition cosmétique conforme à l'invention, sous forme d'une crème de jour, comprend les ingrédients suivants, dans les quantités en poids suivantes :

| | |
|---|---|
| Eau purifiée | QSP 100 |
| Principe actif E5 | 2 à 7 % |
| Acétate de Vitamine E | 0,2 à 1 % |
| Phytosqualane | 1 à 5 % |
| Beurre de karité | 1 à 3 % |
| Glycérine | 5 à 10 % |
| Huile de macadamia | 1 à 2 % |
| Triglycérides capriques/capryliques | 0,5 à 2 % |
| Parfum | 0,1 % |

Cette composition est destinée à être appliquée par voie topique sur la peau du visage et du cou, à raison d'une fois par jour, par exemple le matin.

Elle présente un effet antivieillissement cutané important.

**Revendications**

1. Principe actif cosmétique ou pharmaceutique, **caractérisé en ce qu'**il comprend un extrait végétal obtenu par extraction d'un mélange de matières premières végétales contenant de l'écorce de chêne, du bois de chêne et des feuilles de chêne, ledit mélange contenant les concentrations suivantes, exprimées en masse par rapport à la masse totale dudit mélange :

    - 5 à 15 % de feuilles de chêne,

13

- 5 à 15 % de bois de chêne,
- et 70 à 90 % d'écorce de chêne.

2. Principe actif selon la revendication 1, dans lequel ledit mélange de matières premières végétales contient en outre des bourgeons de chêne.

3. Principe actif selon l'une quelconque des revendications 1 à 2, dans lequel ledit extrait végétal est un extrait aqueux.

4. Principe actif selon l'une quelconque des revendications 1 à 3, dans lequel ledit extrait végétal est un extrait susceptible d'être obtenu par extraction dudit mélange de matières premières végétales par un véhicule d'extraction contenant de l'eau et de la glycérine.

5. Principe actif selon l'une quelconque des revendications 1 à 4, dans lequel ledit mélange de matières premières végétales est issu de chêne appartenant à une espèce choisie parmi *Quercus robur, Quercus petraea* et *Quercus x rosacea,* ou d'un mélange de chênes de telles espèces.

6. Utilisation non-thérapeutique d'un principe actif selon l'une quelconque des revendications 1 à 5 dans le domaine cosmétique, pour lutter contre le vieillissement cutané.

7. Composition cosmétique ou dermatologique ou pharmaceutique à usage topique, comportant un principe actif selon l'une quelconque des revendications 1 à 5 dans un excipient cosmétiquement ou pharmaceutiquement compatible.

8. Composition selon la revendication 7, dans laquelle la teneur en ledit principe actif est comprise entre 0,1 et 10 % en poids par rapport au poids total de la composition.

9. Composition selon la revendication 8, dans laquelle la teneur en ledit principe actif est comprise entre 0,1 et 7 % en poids par rapport au poids total de la composition.

10. Composition selon la revendication 7, dans laquelle la teneur en ledit principe actif est comprise entre 0,1 et 5 % en poids par rapport au poids total de la composition.

11. Composition selon la revendication 10, dans laquelle la teneur en ledit principe actif est comprise entre 0,1 et 2 % en poids par rapport au poids total de la composition.

12. Composition selon la revendication 7, dans laquelle la teneur en ledit principe actif est comprise entre 2 et 7 % en poids par rapport au poids total de la composition.

13. Procédé de traitement cosmétique non-thérapeutique de la peau, comprenant l'application par voie topique d'une composition selon l'une quelconque des revendications 7 à 12.

**Patentansprüche**

1. Kosmetischer oder pharmazeutischer Wirkstoff, **dadurch gekennzeichnet, dass** er einen Pflanzenextrakt umfasst, der durch Extraktion einer Mischung von pflanzlichen Ausgangsmaterialien, die Eichenrinde, Eichenholz und Eichenblätter enthält, erhalten wird, wobei die Mischung die folgenden Konzentrationen enthält, die als Gewicht in Bezug auf das Gesamtgewicht der Mischung ausgedrückt werden:

   - 5 bis 15% Eichenblätter,
   - 5 bis 15% Eichenholz,
   - sowie 70 bis 90% Eichenrinde.

2. Wirkstoff nach Anspruch 1, wobei die Mischung aus pflanzlichen Ausgangsmaterialien weiterhin Eichenknospen enthält.

3. Wirkstoff nach einem der Ansprüche 1 bis 2, wobei es sich bei dem Pflanzenextrakt um einen wässrigen Extrakt handelt.

**4.** Wirkstoff nach einem der Ansprüche 1 bis 3, wobei es sich bei dem Pflanzenextrakt um einen Extrakt, der durch Extraktion der Mischung von pflanzlichen Ausgangsmaterialien mit einem Extraktionsmittel, das Wasser und Glycerin enthält, erhältlich ist, handelt.

**5.** Wirkstoff nach einem der Ansprüche 1 bis 4, wobei die Mischung von pflanzlichen Ausgangsmaterialien aus Eiche, die zu einer Spezies ausgewählt aus *Quercus robur, Quercus petraea* und *Quercus x rosacea* oder aus einer Mischung von Eichen dieser Spezies gehört, stammt.

**6.** Nichttherapeutische Verwendung eines Wirkstoffs nach einem der Ansprüche 1 bis 5 auf dem Gebiet der Kosmetik zur Bekämpfung der Hautalterung.

**7.** Kosmetische oder dermatologische oder pharmazeutische Zusammensetzung für die topische Verwendung, umfassend einen Wirkstoff nach einem der Ansprüche 1 bis 5 in einem kosmetisch oder pharmazeutisch unbedenklichen Grundstoff.

**8.** Zusammensetzung nach Anspruch 7, in der der Wirkstoffgehalt zwischen 0,1 und 10 Gew.-% in Bezug auf das Gesamtgewicht der Zusammensetzung liegt.

**9.** Zusammensetzung nach Anspruch 8, in der der Wirkstoffgehalt zwischen 0,1 und 7 Gew.-% in Bezug auf das Gesamtgewicht der Zusammensetzung liegt.

**10.** Zusammensetzung nach Anspruch 7, in der der Wirkstoffgehalt zwischen 0,1 und 5 Gew.-% in Bezug auf das Gesamtgewicht der Zusammensetzung liegt.

**11.** Zusammensetzung nach Anspruch 10, in der der Wirkstoffgehalt zwischen 0,1 und 2 Gew.-% in Bezug auf das Gesamtgewicht der Zusammensetzung liegt.

**12.** Zusammensetzung nach Anspruch 7, in der der Wirkstoffgehalt zwischen 2 und 7 Ges.-% in Bezug auf das Gesamtgewicht der Zusammensetzung liegt.

**13.** Verfahren zur kosmetischen nichttherapeutischen Behandlung der Haut, bei dem man eine Zusammensetzung nach einem der Ansprüche 7 bis 12 topisch appliziert.

**Claims**

**1.** Cosmetic or pharmaceutical active ingredient, **characterized in that** it comprises a plant extract obtained by extraction of a mixture of plant raw materials containing oak bark, oak wood and oak leaves, said mixture containing the following concentrations, expressed by weight relative to the total weight of said mixture:

- 5% to 15% of oak leaves,
- 5% to 15% of oak wood,
- and 70% to 90% of oak bark.

**2.** Active ingredient according to Claim 1, in which said mixture of plant raw materials also contains oak buds.

**3.** Active ingredient according to either one of Claims 1 and 2, in which said plant extract is an aqueous extract.

**4.** Active ingredient according to any one of Claims 1 to 3, in which said plant extract is an extract that can be obtained by extraction of said mixture of plant raw materials with an extraction carrier containing water and glycerol.

**5.** Active ingredient according to any one of Claims 1 to 4, in which said mixture of plant raw materials is derived from oak belonging to a species chosen from *Quercus robur, Quercus petraea* and *Quercus x rosacea,* or a mixture of oaks of such species.

**6.** Non-therapeutic use of an active ingredient according to any one of Claims 1 to 5, in the cosmetics field, for combatting skin ageing.

7. Cosmetic or dermatological or pharmaceutical composition for topical use, comprising an active ingredient according to any one of Claims 1 to 5 in a cosmetically or pharmaceutically compatible excipient.

8. Composition according to Claim 7, in which the content of said active ingredient is between 0.1% and 10% by weight relative to the total weight of the composition.

9. Composition according to Claim 8, in which the content of said active ingredient is between 0.1% and 7% by weight relative to the total weight of the composition.

10. Composition according to Claim 7, in which the content of said active ingredient is between 0.1% and 5% by weight relative to the total weight of the composition.

11. Composition according to Claim 10, in which the content of said active ingredient is between 0.1% and 2% by weight relative to the total weight of the composition.

12. Composition according to Claim 7, in which the content of said active ingredient is between 2% and 7% by weight relative to the total weight of the composition.

13. Process for non-therapeutic cosmetic treatment of the skin, comprising the topical application of a composition according to any one of Claims 7 to 12.

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP 2006249018 A
- KR 20080097314

**Littérature non-brevet citée dans la description**

- **ALMEIDA et al.** *Biofactors,* 2008, vol. 33, 267-279
- **BLOIS.** *Nature,* 1958, vol. 181, 1199-1200
- **DUDONNÉ et al.** *J. Agric. Food Chem.,* 2011, vol. 59, 4527-4536
- *Anal Biochem,* 1997, vol. 253, 162
- *J Immunol Methods,* 1997, vol. 202, 133
- *J Neurochem,* 2001, vol. 79, 266
- *Am. J Physiol Lung Cell Mol Physiol,* 2001, vol. 281, L993
- *Mol Hum Reprod,* 2001, vol. 7, 237
- *Anal Biochem,* 2000, vol. 287, 196
- *J Invest Dermatol,* 1999, vol. 112, 751
- **SIM et al.** *Arch Pharm Res,* 2007, vol. 30 (3), 290-298
- **LEE et al.** *Annals of the New York Academy of Sciences,* 1999, vol. 878, 635-637